# EUROPEAN PATENT SPECIFICATION

(11) **EP 3 072 874 B1**
(45) Date of publication and mention of the grant of the patent: **29.05.2019**
(21) Application number: 14863702.8
(22) Date of filing: 18.11.2014
(51) Int. Cl.: C07C 37/54, C07C 39/04, C07C 39/07, C07C 39/08, C07C 45/59, C07C 47/58, C07C 41/01, C07C 43/23, C07C 29/00, C07G 1/00

(54) **NOVEL METHOD FOR THE DEPOLYMERISATION OF LIGNIN**
NEUARTIGES VERFAHREN ZUR DEPOLYMERISIERUNG VON LIGNIN
NOUVEAU PROCÉDÉ DE DÉPOLYMÉRISATION DE LA LIGNINE

(30) Priority: 22.11.2013 ES 201331705
(43) Date of publication of application: 28.09.2016
(73) Proprietor: Universidad Del Pais Vasco Euskal Herriko Unibertsitatea, 48940 Leioa (Vizcaya) (ES)
(72) Inventor: ERDOCIA IRIARTE, Xabier, E-48940 Leioa (Viszcaya) (ES); CORCUERA MAESO, Mª de los Ángeles, E-48940 Leioa (Viszcaya) (ES); LABIDI BOUCHRIKA, Jalel, E-48940 Leioa (Viszcaya) (ES)
(74) Representative: Balder IP Law, S.L.
(86) International application number: PCT/ES2014/070854
(87) International publication number: WO 2015/075290

(56) References cited:
- US-A1- 2012 168 101
- ERDOCIA, X ET AL.: 'Organosolv black liquor hydrolysis to obtain low molecular weight phenolic compounds' CHEMICAL ENGINEERING , 2012 vol. 29, 2012, XP008183266
- TOLEDANO, A. ET AL.: 'Process for olive tree pruning lignin revalorisation' CHEMICAL ENGINEERING JOURNAL, 2012 vol. 193, 15 June 2012, pages 396 - 403, XP028493155
- TOLEDANO, A. ET AL.: 'Organosolv lignin depolymerization with different base catalysts' JOURNAL OF CHEMICAL TECHNOLOGY AND BIOTECHNOLOGY, 2012 vol. 87, no. 11, 03 April 2012, pages 1593 - 1599, XP055343941

## Description

### Field of the invention

The present invention relates to a novel method for the depolymerization of lignin which starts from a black liquor which contains it obtained from the delignification of the biomass.

### Background of the invention

The current methods for degrading lignin in the state of the art are diverse and although they use a wide range of operating methods and conditions, they all have similar disadvantages. There are numerous processes which allow for the degradation of lignin but all of them have some problems which limit the use thereof on an industrial scale. Amongst other things, due to inadequate adaptation to the industrial needs and/or incompatibility with the increasingly stricter environmental requirements.

Among these methods for the depolymerization of lignin, the following can be cited as an example: the method described in US 5,807,952 based on pyrolysis; a solvothermal method described in US5,959,167; methods which use catalysts based on transition metals (US 2,220,624), a method in the presence of melted salts of a hydroxide of general formula M(OH)n or a mixture of alkaline or alkaline earth hydroxides (US 20130066116) or a hydrothermal method in which the lignin is subjected in a basic medium to pressure and temperature.

A. Toledano, et al. (Journal of Chemical Technology and Biotechnology, 2012, 87(11), 1593-1599) describe a process for the alkaline treatment of lignin derived from olive tree prunings (by organosolve treatment) followed by base-catalyzed depolymerization, acidification, filtration to remove residual lignin and coke, and extraction.

All these current methods of depolymerization of lignin, which allow the revaluation thereof and obtaining of phenolic products with high added-value, nevertheless, have disadvantages, of which one is common to all; they depart from the precipitated solid lignin. The solid lignin is obtained from biomass in general which is subjected to a pasting step, from which a black liquor (or black lye) to which is added an acid which causes the precipitation of the lignin. The lignin precipitate is isolated and the resulting precipitated solid lignin is subjected to a depolymerization method.

In order to precipitate the lignin it is necessary to carry out a series of process steps which extend the global method of the treatment of biomass to obtain aromatic compounds. Furthermore, the precipitation of the lignin comprises the use of additional elements such as filters and reagents which obviously make the method more expensive as a whole and complicate it.

In view of what has been stated, there is still a need to provide a simpler, more efficient and economic method which is scalable to an industrial level which provides aromatic compounds with high yields, overcoming at least part of the disadvantages of the methods of the current state of the art.

### Description of the invention

**Figure 1****:** schematic depiction of an embodiment of the method for obtaining a black liquor with depolymerized lignin (9) from biomass (1) (for example: almond shell)
**Figure 2****:** schematic depiction of the treatment of depolymerized lignin (9) for obtaining the phenolic products (18).
**Figure 3****:** represents the chromatograph obtained by means of gas chromatography mass spectrometry (GC-MS) analysis of oil obtained in Example 1 with NaOH.
**Figure 4****:** represents the chromatograph obtained by means of gas chromatography mass spectrometry (GC-MS) analysis of oil obtained in Example 1 with KOH.

### Description of the invention

In a first aspect, the invention relates to a method for the depolymerization of lignin comprising the following steps:
(i) Providing a black liquor obtained from the alkaline treatment of the delignification of the biomass;
(ii) Concentrating said black liquor until the initial volume of the same is reduced to half;
(iii) Subjecting the concentrated black liquor to hydrothermal treatment;
(iv) Adding an acid to the reaction mixture obtained in the previous step;
(v) Separating the precipitate from the liquid phase resulting in the previous step; and
(vi) Extracting, from the recovered liquid phase, the products of depolymerization of lignin.

The type of biomass which can be subjected to this method, hereinafter the method of the invention, is lignocellulosic biomass which contains, amongst other polymers, lignin. Lignin is a phenolic macromolecule, the structure of which is still not perfectly elucidated. It is present between the cell walls of numerous plants, specifically wood, to which they give rigidity. The biomass usable in the present invention can be derived from wood, green residues in general or straw for example. In one particular embodiment, it comes from the shell of dried fruits, such as the shell of almond.

Lignin is available in the black liquors which result as by-products of the delignification treatments of the biomass, for example, that which takes place during the production of paper to obtain cellulose (paper paste) which generates these by-products also called black lyes or from the production of second generation bioethanol.

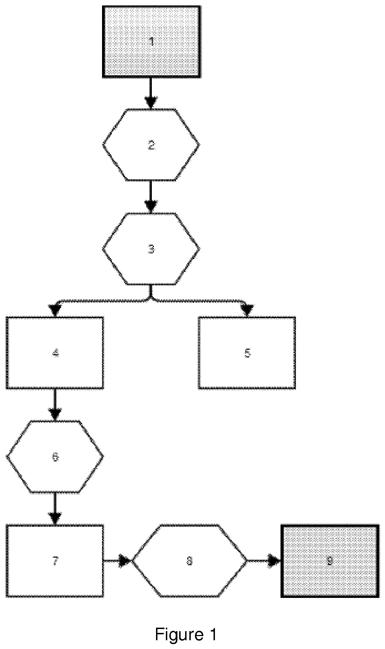

The delignification of the biomass is a process known in the state of the art which consists of carrying out an alkaline treatment of the biomass, by preferably using sodium hydroxide or potassium hydroxide in a typical concentration of 15% by weight of base with respect to the weight of the solution used in the delignification (see Figure 1, where (1) represents the biomass, (2) the step of delignification). In the example of the present invention, a ratio of solid to liquid of 1:10 by weight is used for example and is carried out in an autoclave at 121 °C.

The product which is produced from the delignification is generally firstly filtered with a metallic mesh and then a cloth filter to separate any solid residue from the liquid phase where the lignin is dissolved (black liquor) (see Figure 1, (3) where this filtering step is depicted schematically, (5) the delignified paste, and (4) the resulting black liquor).

In the step (ii), the black liquor (4) is concentrated by means of any conventional technique (Figure 1, (6)). In one particular embodiment, it is carried out by evaporation either at atmospheric pressure or applying a vacuum and with temperature, for example using a rotary evaporator with a water bath. Typically the concentration is carried out at 60 °C and vacuum of 0.02 MPa.

The concentration of the black liquor (4) is carried out until the initial volume of the same is reduced to half (see (7) in Figure 1, which represents the concentrated liquor). Preferably it should be carried out avoiding the lignin precipitating. In this context, the inventors have observed that a very good yield of the depolymerization is thereby obtained and in addition excessive consumption of energy is avoided in concentrating the black liquor even further which only entails a small additional improvement to the yield.

The hydrothermal treatment to which the black liquor is subjected is a conventional treatment well known by a person skilled in the art. During the treatment, the depolymerization of the lignin takes place. This step (iii) is depicted in Figure 1 by means of the element (8) after which (9) is obtained: a reaction mixture. This treatment is generally carried out in the presence of a base, a temperature between 250 to 500 °C and high pressure, generally between 5-30 MPa. In one preferred embodiment according to the present invention, the hydrothermal treatment is carried out at 300 °C, 9 MPa and under continuous agitation. During this treatment, the lignin is depolymerized and a reaction mixture is obtained which contains the depolymerized lignin from which it is possible to separate the products obtained. The depolymerization times are usually between 30 minutes and 2 hours depending, for example on the depolymerization conditions.

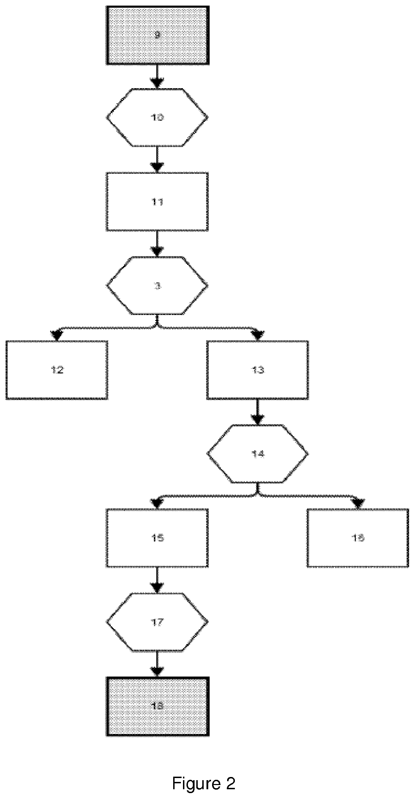

In order to separate the products obtained which are in the reaction mixture (Figure 1, (9) and Figure 2 (9)) resulting from step (iii), an acid, such as hydrochloric acid, sulfuric acid or a mixture of both is added to the reaction mixture (Figure 2 (10)). It is added in a quantity which allows it to reach a pH value of 1. At this pH value, the inventors have observed that the total precipitation of all the residue formed in the depolymerization reaction is achieved. The pH can be adjusted to a value greater than 1, but in this case the subsequent liquid-liquid extraction is difficult since it is has been observed that a precipitate appears.

The step of separating the precipitate from the liquid phase is then carried out, for example by means of filtration, although any other conventional separation means can be used. In one particular embodiment, the filtration is carried out using a filter with a pore diameter of 7-12 µm. In Figure 2, (11) represents the acidified reaction mixture, (3) the filtering step, (12) the precipitate and (13) the liquid phase.

The liquid phase (13) separated in the previous step is subjected to an extraction step of the depolymerization products (14). The extraction produces an aqueous phase (16) and an organic phase (15). The extraction is preferably carried out with ethyl acetate since it is the most appropriate product for dissolving the monomers formed, although other organic solvents can also be used such as diethyl ether, vinyl acetate or n-Butyl acetate.

The method of the invention also comprises recovering the depolymerization products.

The recovery is carried out by means of the steps of: (a) evaporating the solvent of the organic liquid phase resulting from the extraction; (b) resuspending the organic oil resulting from step (a) in an organic solvent; and (c) subjecting the resulting suspension to separation by chromatography.

The evaporation (depicted in (17), Figure 2) is carried out for example in a rotary evaporator at a typical temperature of 50 °C and in a vacuum (for example at 0.02 MPa) until the complete elimination of the solvent used in the liquid-liquid extraction. The resulting isolated oil (without solvent) (18) is weighed to find out the yield and is resuspended (dissolved) in the same solvent used in the extraction, preferably in ethyl acetate, [but with high performance liquid chromatography (HPLC)]. The suspension is then subjected to chromatography, for example gas chromatography in order to separate the compounds present.

These compounds are generally aromatic and they are essentially of the family of phenols. Among the compounds that can be obtained, in the form of a mixture, there are phenol, cresols, catechol and the derivatives thereof (3-methylcatechol, 4-methylcatechol and 4-ethylcatechol).

Figure 1 schematically depicts the steps of the method of the invention (i) to (iii). The biomass is situated in (1) and in (2) it is subjected to delignification alkaline treatment previously described. The resulting product is filtered in (3) producing delignified paste (5) and a black liquor (4). The black liquor is concentrated according to step (ii) of the method of the invention in (6) and then the concentrated liquor (7) is subjected to the depolymerization (step (iii) of the method of the invention), depicted in (8) to give the reaction mixture depicted in (9).

Figure 2 depicts the continuation of the method where the reaction mixture (9) is acidified according to step (iv) of the method of the invention in (10) to yield an acidified mixture (11) which is subjected to a filtering step (3) to produce a precipitated solid (12) and a liquid phase (13) separated according to step (v) of the method of the invention. The liquid-liquid extraction, step (vi) of the method of the invention, is depicted as (14) produces an aqueous phase (16) and an organic phase (15). From this phase, for example, by evaporating the solvent in (17), an oil is obtained with the depolymerization products (18).

The method of the invention is more economic and more interesting from an environmental point of view due to what has been previously mentioned. It allows an industrial by-product, the black lye, to be disposed of and the revaluation thereof by means of obtaining a mixture of organic compounds of high added-value which can be used in numerous industries and applications.

### Examples

### Example 1

50 grams of almond shell were triturated into a Retsch 2000 hammer mill to produce chips of 4-6 mm free of small stones, powder and earth.

The 50 grams of milled almond shell were then introduced into a 1 liter recipient implemented in borosilicate 3.3 glass according to the standard DIN EN ISO 4796-1 together with 500 grams of a solution of 15% by weight of KOH. The delignification process was carried out in an autoclave for 90 mins, at a temperature of 121 °C and a pressure of 0.2 MPa.

After the delignification process, the reaction mixture was firstly filtered with a metallic mesh (hole size of 2 mm) and subsequently with a cloth filter to separate the solid residue from the black liquor where the lignin was dissolved. After this, the black liquor was concentrated until the volume thereof was reduced to half, using a rotary evaporator, at 60 °C in a vacuum atmosphere (0.02 MPa). Once the liquor was concentrated, the depolymerization process was carried out.

The depolymerization process was carried out in the following manner. 30 grams of concentrated liquor were introduced into a steel reactor under pressure. The conditions were: 300 °C, 80 mins, 9 M Pa and continuous agitation. Once the depolymerization process of the lignin contained in the black liquor had been carried out, the products were then separated.

To this end, the reaction mixture obtained after the depolymerization process was acidified with HCl until a pH of 1 was reached. When this addition of acid was carried out, a precipitate (residue) was produced which was eliminated by filtration with a filter paper with a pore diameter of 7-12 µm. A liquid-liquid extraction was carried out with ethyl acetate at the liquid phase, where the depolymerization products of the lignin were found. This extraction process was carried out until, upon adding the ethyl acetate, no change was observed in the color in the solvent, that is to say, no more products were extracted from the aqueous phase. After said extraction process, the phenolic products derived from the lignin were in the organic phase. This organic phase was subjected to an evaporation process to eliminate the solvent and isolate the depolymerization products of the lignin. The evaporation was carried out in a rotary evaporator at 50 °C and in a vacuum atmosphe re (0.02 MPa).

Once the entire solvent (ethyl acetate) had been eliminated, the evaporation was concluded and 0.337 grams of an oil were obtained which contained the depolymerization compounds of the lignin.

The specific results for the case described are shown below and for the case using 15% by weight of NaOH instead of KOH.

The calculations of the oil obtained were carried out with respect to three parameters: lignin contained in the black liquor, organic material contained in the black liquor and with respect to the total of the black liquor.

**Table 1. Yield of the oil with the depolymerization products of the lignin (% by weight).**

| | **Oil/lignin** | **Oil/organic material** | **Oil/black liquor** |
|---|---|---|---|
| NaOH | 54.5052 | 12.6458 | 0.8612 |
| KOH | 71.0224 | 20.3289 | 1.1222 |

The products generated after the direct depolymerization of the lignin have been identified by means of gas chromatography mass spectrometry (GC-MS). The most representative compounds and those which have also been quantified are shown in the following table:

**Table 2. Percentage by weight with respect to the oil of the monomeric units of phenolic compounds obtained**

| **Compound (%)** | **NaOH** | **KOH** |
|---|---|---|
| Phenol | 0.31 | 0.64 |
| Cresols | 0.38 | 0.40 |
| Guaiacol | 0.03 | 0.19 |
| Catechol | 1.51 | 0.99 |
| 4-methylcatechol | 0.20 | 0.23 |
| Syringol | | 0.27 |
| 4-hydroxybenzaldehyde | | 0.05 |
| Vanillin | | 0.20 |
| Acetovanillone | | 0.07 |
| 4-hydroxy-3-methoxy-phenyl acetone | | 0.02 |
| 4-ethylcatechol | 0.23 | |

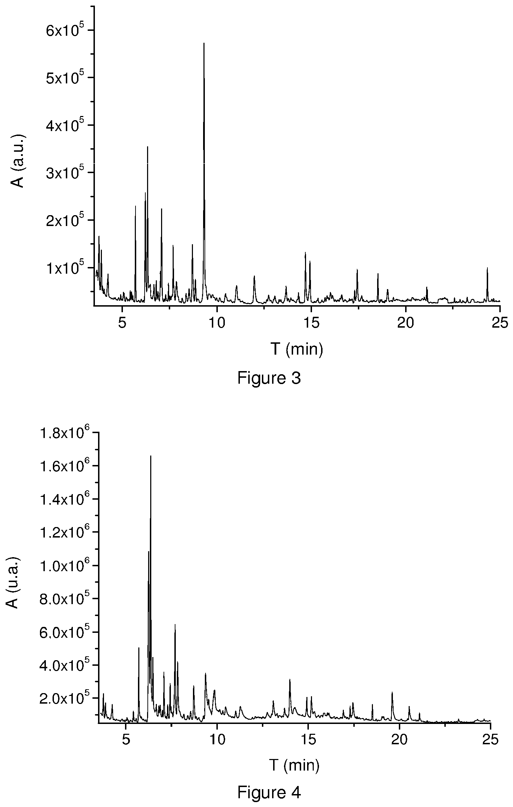

In Figure 3, the chromatograph obtained by means of GS-MS of the analysis of the oil produced after the depolymerization of the lignin with NaOH is shown and in Figure 4 with KOH.

The invention is not limited to the specific embodiments which have been described, but rather also encompass, for example the variants which can be carried out by the person skilled in the art within the scope of the claims.

## Claims

1. A method for the depolymerization of lignin comprising the following steps:
(i) Providing a black liquor obtained from the alkaline treatment of the delignification of the biomass;
(ii) Concentrating said black liquor until the initial volume of the same is reduced to half;
(iii) Subjecting the concentrated black liquor to hydrothermal treatment;
(iv) Adding an acid to the reaction mixture obtained in the previous step;
(v) Separating the precipitate from the liquid phase resulting in the previous step; and
(vi) Extracting, from the recovered liquid phase, the products of depolymerization of lignin.

2. The method according to claim 1, wherein the concentration is carried out at 60 °C and in a vacuum of 0.02 MPa.

3. The method according to any one of claims 1 to 2, wherein the hydrothermal treatment is carried out at 300 °C, 9 MPa and under continuous agitation.

4. The method according to any one of claims 1 to 3, wherein the acid which is added in step (iv) is hydrochloric acid, sulfuric acid or a mixture.

5. The method according to claim 4, wherein the quantity of acid added is that required to adjust the pH to 1.

6. The method according to any one of claims 1 to 5, wherein the separation of the precipitate from the liquid phase is carried out by filtration.

7. The method according to any one of claims 1 to 6, wherein the extraction of the depolymerization products is carried out with ethyl acetate.

8. The method according to any one of claims 1 to 7, which also comprises recovering the depolymerization products.

9. The method according to claim 8, wherein the recovery comprises carrying out the following steps: (a) evaporating the solvent of the organic liquid phase resulting from the extraction; (b) resuspending the organic oil resulting from step (a) in an organic solvent; and (c) subjecting the resulting suspension to separation by chromatography.

## Patentansprüche

1. Ein Verfahren zur Depolymerisation von Lignin, umfassend die folgenden Schritte:
(i) Zurverfügungstellung einer Schwarzlauge, welche durch alkalische Behandlung der Delignifizierung der Biomasse erhalten wurde;
(ii) Konzentration der Schwarzlauge, bis das ursprüngliche Volumen derselben auf die Hälfte reduziert ist;
(iii) Unterwerfen der konzentrierten Schwarzlauge einer hydrothermalen Behandlung;
(iv) Hinzufügen einer Säure zu dem Reaktionsgemisch aus dem vorherigen Schritt;
(v) Trennung des Präzipitats von der flüssigen Phase erhalten aus dem vorherigen Schritt; und
(vi) Extraktion der Produkte der Depolimerisation von Lignin aus der erhaltenen flüssigen Phase.

2. Das Verfahren nach Anspruch 1, wobei die Konzentration bei 60°C und in einem Vakuum von 0,02 MPa durchgeführt wird.

3. Das Verfahren nach einem der Ansprüche 1 oder 2, wobei die hydrothermale Behandlung bei 300°C, 9 MPa und unter fortwährendem Schütteln durchgeführt wird.

4. Das Verfahren nach einem der Ansprüche 1 bis 3, wobei als Säure in Schritte (iv) Salzsäure, Schwefelsäure oder eine Mischung verwendet wird.

5. Das Verfahren nach Anspruch 4, wobei die Menge der zugesetzten Säure benötigt wird, um den pH auf 1 einzustellen.

6. Das Verfahren nach einem der Ansprüche 1 bis 5, wobei die Trennung des Präzipitats von der flüssigen Phase durch Filtration ausgeführt wird.

7. Das Verfahren nach einem der Ansprüche 1 bis 6, wobei die Extraktion des depolimerisierten Produkts mit Ethylazetat durchgeführt wird.

8. Das Verfahren nach einem der Ansprüche 1 bis 7, welches weiterhin die Gewinnung des depolimerisierten Produkts umfasst.

9. Das Verfahren nach Anspruch 8, wobei die Gewinnung die folgenden Schritte umfasst: (a) Evaporieren des Lösungsmittels aus der flüssigen organischen Phase aus der Extraktion; (b) Wiederaufnahme des organischen Öls resultierend aus Schritt (a) in einem organischen Lösungsmittel; und (c) Unterwerfen der resultierenden Suspension einer Trennung durch Chromatografie.

## Revendications

1. Méthode de dépolymérisation de la lignine comprenant les étapes suivantes :
(i) Fournir une liqueur noire provenant du traitement alcalin de la délignification de la biomasse ;
(ii) Concentrer ladite liqueur noire jusqu'à ce que son volume initial soit réduit de moitié ;
(iii) Soumettre la liqueur noire concentrée à un traitement hydrothermique ;
(iv) Ajouter un acide au mélange réactionnel obtenu à l'étape précédente ;
(v) Séparer le précipité de la phase liquide résultant de l'étape précédente ; et
(vi) Extraire, à partir de la phase liquide récupérée, les produits de dépolymérisation de la lignine.

2. Méthode selon la revendication 1, dans laquelle la concentration est réalisée à 60 °C dans un vide de 0,02 MPa.

3. Méthode selon l'une quelconque des revendications 1 à 2, dans laquelle le traitement hydrothermique est réalisé à 300 °C, 9 MPa et sous agitation continue.

4. Méthode selon l'une quelconque des revendications 1 à 3, dans laquelle l'acide qui est ajouté à l'étape (iv) est un acide chlorhydrique, un acide sulfurique ou un mélange.

5. Méthode selon la revendication 4, dans laquelle la quantité d'acide ajoutée est celle requise pour ajuster le pH à 1.

6. Méthode selon l'une quelconque des revendications 1 à 5, dans laquelle la séparation du précipité de la phase liquide est réalisée par filtration.

7. Méthode selon l'une quelconque des revendications 1 à 6, dans laquelle l'extraction des produits de dépolymérisation est réalisée à l'aide d'acétate d'éthyle.

8. Méthode selon l'une quelconque des revendications 1 à 7, qui comprend également la récupération des produits de dépolymérisation.

9. Méthode selon la revendication 8, dans laquelle la récupération comprend la mise en oeuvre des étapes suivantes : (a) l'évaporation du solvant de la phase liquide organique résultant de l'extraction ; (b) la remise en suspension de l'huile organique résultant de l'étape (a) dans un solvant organique ; et (c) la soumission de la suspension résultante à séparation par chromatographie.
